# EUROPEAN PATENT APPLICATION

(11) **EP 3 181 697 A1**
(43) Date of publication of application: **21.06.2017**
(21) Application number: 15199955.4
(22) Date of filing: 14.12.2015
(51) Int. Cl.: C12Q 1/68

(54) **DETECTING CHOLESTEROL DEFICIENCY MUTATION IN CATTLE**

(71) Applicant: Université de Liège, 4031 Angleur (BE)
(72) Inventor: CHARLIER, Carole, 4000 Liege (BE); GEORGES, Michel, 4000 Liege (BE); HARLAND, Chad, 4000 Liege (BE); COPPIETERS, Wouter, 4000 Liege (BE)

(57) **Abstract**

This invention relates to methods for the detection of bovine that are affected by or carriers of Cholesterol Deficiency (CD). It is based on the identification of a 7 Kb insertion of a BoERVK element in exon 5 of the bovine *APOB* gene that is causing Cholesterol Deficiency The present invention provides methods for determining whether a bovine is affected by or carrier of CD by analyzing its genomic DNA or its RNA.

## Description

### Field of the invention

This invention relates to methods for the detection of bovine that are affected by or carriers of "cholesterol deficiency" (CD), an inherited defect with autosomal recessive inheritance. The present invention provides methods for determining whether a bovine is affected by or carrier of CD by analyzing its genomic DNA or its RNA. The methods include obtaining a sample of material containing genomic DNA or RNA from the bovine, and genotyping said nucleic acid for the presence of a 7 Kb insertion of a bovine endogenous retrovirus sequence (BoERV) in exon 5 of the bovine apolipoprotein B (*APOB*) gene, the mutation causing the Cholesterol Deficiency syndrome.

### Description of the background art

***Marker assisted selection against genetic defects in livestock.*** Intense selection for desired characteristics in livestock often results in increased inbreeding which contributes to the emergence of novel recessive defects. Examples of such outburst in Holstein-Friesian cattle include bovine leucocyte adhesion deficiency (BLAD)(1) and complex vertebral malformation (CVM)(2). Calf mortality resulting from such defects causes important economic losses and raises welfare concerns.
Most inherited defects are autosomal recessive, and are typically due to loss-of function mutations (symbol "*d*") in essential genes. Matings between animals that are healthy but carry one copy of the mutation (genotype "+/*d*", i.e. "carriers") will yield 25% of homozygous mutant animals (genotype *"d*/*d"*) that will be affected. A diagnostic test that allows the identification of +/*d* carrier animals, can be used either to cull carrier animals thereby eliminating the mutation and hence the defect from the population, or to avoid "at risk" matings between carrier sires and dams. The recent development of highly effective genomic tools, now allows for the rapid identification of the causative "*d*" mutations at the molecular level (3). Once identified, effective diagnostic tests can be developed using a range of generic DNA-based technologies that are well known by the people skilled in the art.

***Cholesterol Deficiency and the Cholesterol Deficiency Haplotype*.** Recently, multiple cases of male and female calf mortality following a 1-5 month period of failure of thrive, diarrhea, and pathognomonic low levels of total, HDL and non-HDL cholesterol were reported in Holstein-Friesian cattle (4). All affected animals descended from *Maughlin Storm* on paternal and maternal side, suggesting autosomal recessive inheritance. Accordingly, autozygosity mapping revealed a 2.6Mb BTA11 haplotype (74.5-77.1 Mb), referred to as CDH, for which all cases were homozygous. Lists of bulls carrying the CDH haplotype were published by several breeding companies (CRV (The Netherlands), VIT (Germany), GEN'France, WestGen (Canada),.. ). This indirect haplotype-based test has already been used to detect +/*d* carrier animals. However, because the association between the disease causing "*d*" allele and the SNP alleles is not perfect, such indirect test suffer from a lack of senstivity and specificity. Some homozygous +/+ animals may erroneously be called carriers, while some +/*d* carrier animals may be missed. Improved diagnostic tests, ideally based on the detection of the causative mutation hence having near-perfect sensibility and specificity, were thus needed.

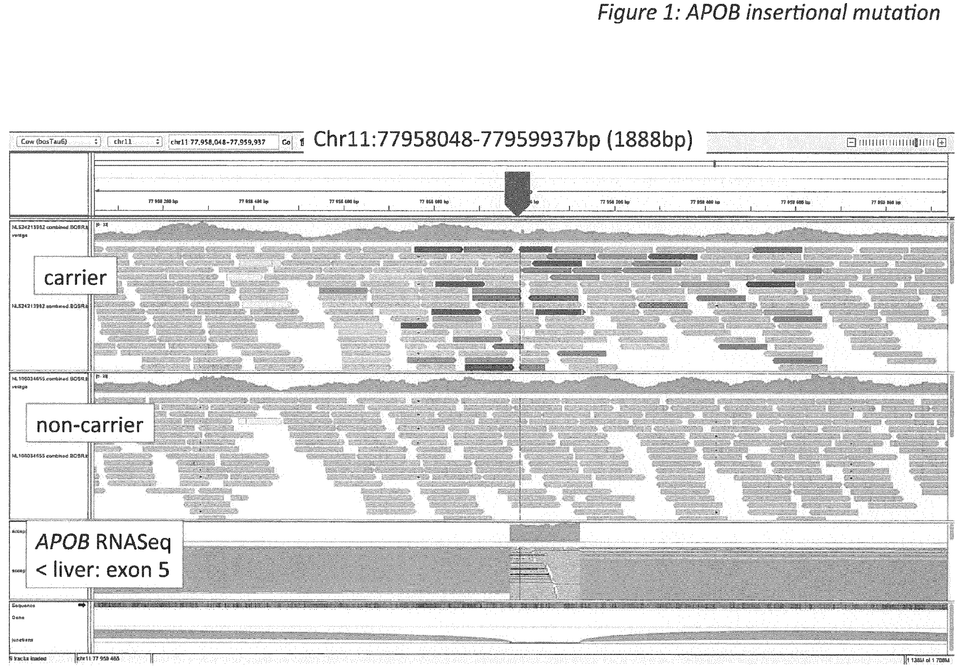

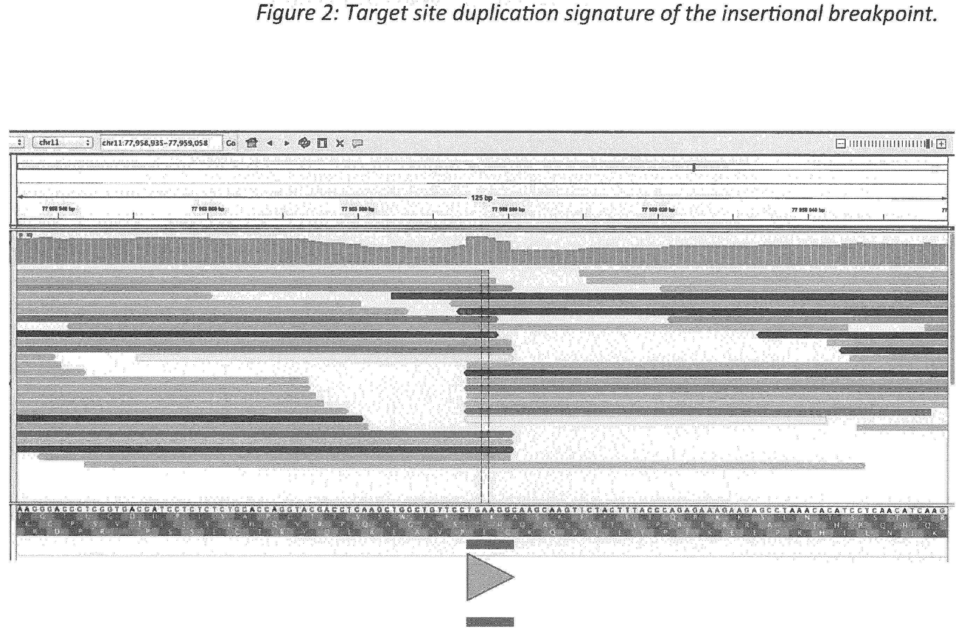

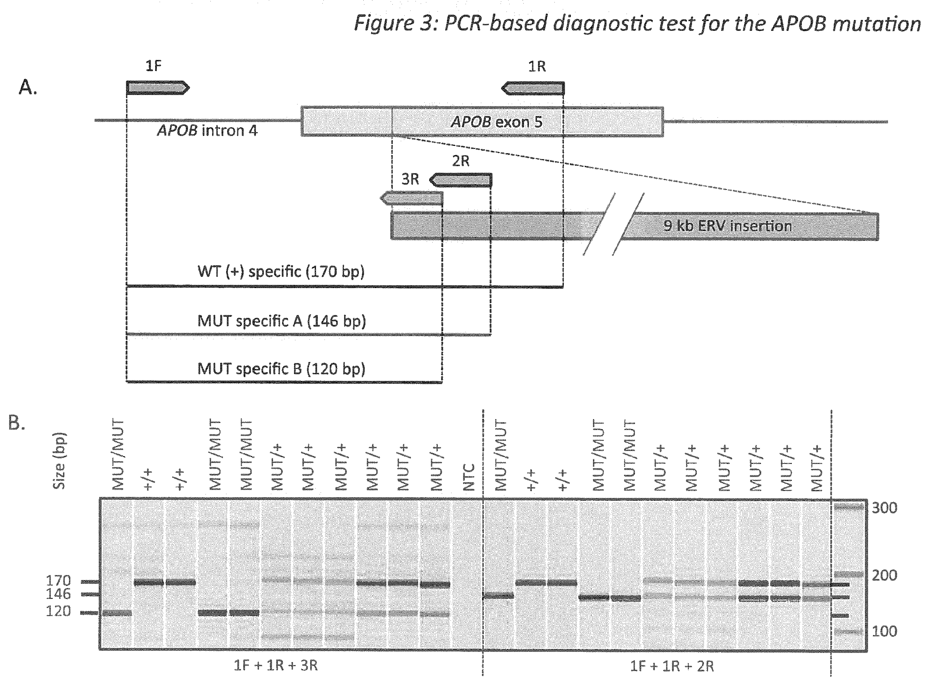

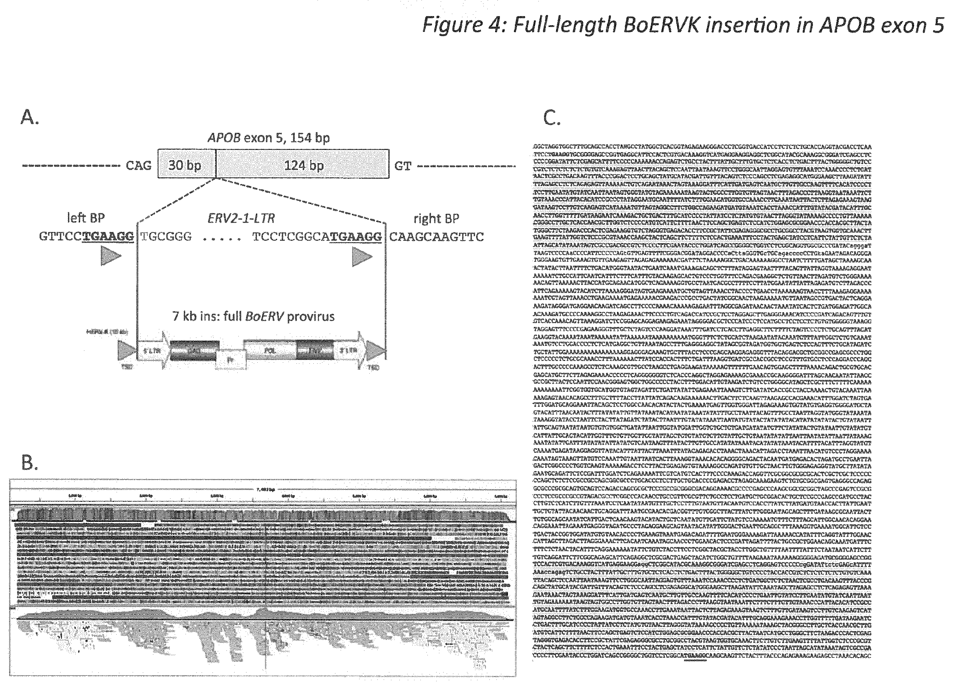

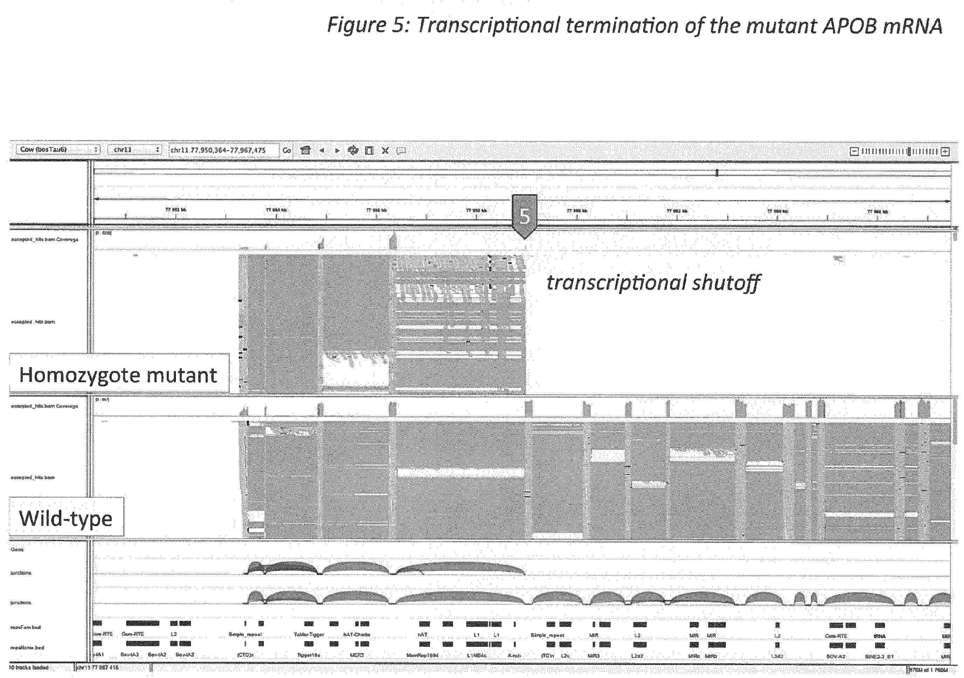

***A polymorphic boERV element that is mobilized in the germline of specific individuals causes cholesterol deficiency in cattle by disrupting the APOB gene.*** The whole genome of four of these carrier bulls had been sequenced at ≥ 20-fold depth as part of the Damona project. The Damona project was primarily designed for the detection of de novo mutations and involves the sequencing of 115 three generation pedigrees comprising sire, dam, offspring sequenced at ≥ 20-fold depth, plus five grand-offspring sequenced at ≥ 4-fold depth. Mining the corresponding dataset in the chr11:74.5-77.1 Mb (bosTau6 reference assembly) interval for disruptive mutations shared heterozygous by the four carrier sires did not reveal any obvious candidate causative gene nor mutation. We noted, however, that the ***APOB*** gene coding for the main apolipoprotein of chylomicrons, very low density lipoproteins (VLDL), and low density lipoproteins (LDL), mapped at position chr11: 77953380-78040118 bp (bosTau6 reference assembly), hence close to the published interval. More than 30 loss-of-function mutations in the ***APOB*** gene have been reported in humans, causing semidominant familial hypolipoproteinemia (FHBL) with - in homozygotes - symptoms that are highly reminiscent of the bovine condition. While no disruptive ***APOB*** mutations shared heterozygous by the four carriers could be predicted by SNP Effect Predictor (5), visual examination of the raw BAM files in the Integrative Genomics Viewer (IGV) identified a cluster of discordant paired reads accounting collectively for ∼50% of sequence depth in exon 5, and pointing towards an LTR element insertion shared by the four carriers as well as 11 more animals in the Damona dataset (Figure 1). All of these would trace back to *Maughlin Storm*, the acknowledged introducer of the CD mutation in Holstein-Friesians (4). We manually assembled two non-overlapping segments (5' and 3' boundaries) of an LTR element from the discordant mates, and used split reads to define the breakpoints at single-base pair resolution, revealing a 6-bp target site duplication typical of retrotransposition events (Figure 2). We developed a PCR-based assay and genotyped ∼700 of the Damona animals as well as ∼500 more recent Dutch Holstein animals (2014-2015) (Figure 3). We confirmed the carrier status of the 15 animals identified from the sequence data and estimated the frequency of the mutation in the Dutch Holstein current population at ∼4%.
To further characterize the LTR element insertion, we performed long range PCR from genomic DNA of two affected calves shown with the genotyping assay to be homozygous mutant. The ensuing gel-purified ∼7Kb amplification product was sequenced by (i) shotgun sequencing on a MiSeq instrument after generating a NexteraXT DNA library (Illumina), complemented by (ii) long single molecule sequencing on a Minion instrument (Oxford Nanopore). The complete sequence of the LTR element was assembled using custom-made scripts, showing that it corresponds to a BoERV-K element (one of the 24 known BoERVs; (6)) (Figure 4). The complete sequence is given in Annex 1. We further extracted total RNA from the livers of the two affected calves and generated strand-specific rRNA-depleted cDNA libraries that were sequenced (2x76bp paired-ends) on a HiSeq 2000 instrument. We analyzed the ensuing 80 million bp of reads with TopHat/Cufflink and visualized the outputs with IGV, revealing complete *APOB* transcriptional shutoff downstream of the LTR insertion (Figure 5). The RNA-Seq results were confirmed by QRT-PCRs targeting upstream (3-4) as well as downstream exons (6-7 and 7-8). Provided that the truncated mRNA remains translatable, the encoded protein would only represent <3% of the full-length protein.
Taken together, these findings provide very strong support that the insertion of this BoERV-K element in the 5th exon of the *APOB* gene (hereafter referred to as CD mutation) causes Cholesterol Deficiency.

### Summary of the invention

In view of the above, the technical problem underlying the present invention was to provide means and methods that allow for a selective and convenient diagnosis of Cholesterol Deficiency or of carrier-status for this disease in cattle, on the basis of the direct detection on the CD mutation. The solution to said technical problem is achieved by the embodiments characterized in the claims. The present invention provides for the first time the identity of the mutation causing the CD syndrome in cattle.

Thus, the present invention relates in a first embodiment to a method for determining whether a bovine is affected by the Cholesterol Deficiency syndrome or a carrier of Cholesterol Deficiency mutation by analyzing its genomic DNA, the method comprising the steps of:
a. obtaining a sample of material containing said genomic DNA from the bovine,
b. extracting the DNA from said sample,
c. genotyping said DNA for the CD mutation, and
d. determining whether said animal carries the CD mutation.

The term "bovine" in accordance with the present invention encompasses all cattle or cattle breeds from the species Bos taurus. In a preferred embodiment of the methods of the present invention the bovine is selected from the group consisting of Holstein, Friesian and Holstein-Friesian Cross breeds, British and /or Dutch Friesian.

The term "carrier of Cholesterol Deficiency " refers to a bovine that carries the mutation causing the Cholesterol Deficiency defect on one of its chromosomes (whether inherited from sire or dam), and a wild-type allele on the other chromosome.

The term "sample" or "biological sample" according to the present invention refers to any material containing nuclear DNA from said bovine to be tested. In a preferred embodiment the biological sample to be used in the methods of the present invention is selected from the group consisting of blood, sperm, hair roots, milk, body fluids as well as tissues including nucleated cells.
DNA extraction / isolation and purification methods are well-known in the art and can be applied in the present invention. Standard protocols for the isolation of genomic DNA are inter alia referred to in Sambrook, J., Russell, D.W., Molecular Cloning: A Laboratory Manual, the third edition, Cold Spring Harbor Laboratory Press, Cold Spring Harbor, New York, 1.31-1.38, 2001 and Sharma, R.C., et al., A rapid procedure for isolation of RNA-free genomic DNA from mammalian cells, BioTechniques, 14, 176-178, 1993.

The term "CD mutation" refers to insertion of the 6770 base pairs BoERVK element in exon 5 of the *APOB* gene (77,958,495 bp) on bovine chromosome 11. The CD mutation causes premature transcription termination of the APOB gene, generating at best a truncated protein representing ∼3% of the full-length APOB. The term "genotyping said DNA for the CD mutation" in accordance with the present invention refers to a method for determining or identifying whether a particular nucleotide sequence is present in a DNA sample. There are several methods known by those skilled in the art, e.g. (7) for determining whether such nucleotide sequence is present in a DNA sample. These include the amplification of a DNA segment encompassing the mutation by means of the polymerase chain reaction (PCR) or any other amplification method, and interrogate the amplicons by means of allele specific hybridization, or the 3'exonuclease assay (Taqman assay), or fluorescent dye and quenching agent-based PCR assay, or the use of allele-specific restriction enzymes (RFLP-based techniques), or direct sequencing, or the oligonucleotide ligation assay (OLA), or pyrosequencing, or the invader assay, or minisequencing, or DHPLC-based techniques, or single strand conformational polymorphism (SSCP), or allele-specific PCR, or denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, extension based assays, ARMS (Amplification Refractory Mutation System), ALEX (Amplification Refractory Mutation Linear Extension), SBCE (Single base chain extension), a molecular beacon assay, invader (Third wave technologies), a ligase chain reaction assay, 5'-nuclease assay-based techniques, hybridization capillary array electrophoresis (CAE), protein truncation assay (PTT), immunoassays and solid phase hybridization (dot blot, reverse dot blot, chips). This list of methods is not meant to be exclusive, but just to illustrate the diversity of available methods. Some of these methods can be performed in accordance with the methods of the present invention in microarray format (microchips) or on beads.
The invention thus also relates to the use of primers or primer pairs, wherein the primers or primer pairs hybridize(s) under stringent conditions to the DNA corresponding to the CD mutation (Figures 3 & 4), or to the complementary strand thereof.
Preferably, the primers of the invention have a length of at least 14 nucleotides such as 17 or 21 nucleotides.

In one embodiment of the diagnostic test, three primer sets are simultaneously used to respectively amplify the wild-type and the mutant allele. The corresponding amplicons are respectively detected by gel electrophoresis. "Stringent or highly stringent conditions" of hybridization are well known to or can be established by the person skilled in the art according to conventional protocols. Appropriate stringent conditions for each sequence may be established on the basis of well-known parameters such as temperature, composition of the nucleic acid molecules, salt conditions etc.: see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Typical (highly stringent) conditions comprise hybridization at 65°C in 0.5xSSC and 0.1% SDS or hybridization at 42°C in 50% formamide, 4xSSC and 0.1% SDS. Hybridization is usually followed by washing to remove unspecific signals. Washing conditions include conditions such as 65°C, 0.2xSSC and 0.1% SDS or 2xSSC and 0,1% SDS or 0,3xSSC and 0,1% SDS at 25°C - 65°C.

The term "base position 77,958,495 on bovine chromosome 11" refers to the Bos taurus reference sequence (bosTau6) which can be retrieved from e.g. the UCSC, Ensembl, and NCBI genome browsers. The bosTau6 was generated by the Atlas genome assembly system at Baylor College of Medicine Human Genome Sequencing Center. The sequencing strategy combined BAC shotgun reads with whole genome shotgun reads from small insert libraries as well as BAC end sequences.

In a further embodiment of the present invention a method is provided for determining whether a bovine is affected with the Cholesterol Deficiency syndrome or a carrier of Cholesterol Deficiency by analyzing its RNA, the method comprising the steps of :
a. obtaining a sample of material containing said RNA from the bovine,
b. extracting the RNA from said sample,
c. genotyping said RNA for the CD mutation, and
d. ascertaining whether said animal carries the CD mutation.

"RNA" as referred to herein encompasses all types of RNA. Techniques well known in the art in order to allow for the isolation of total RNA, mitochondrial RNA, or messenger RNA. The person skilled in the art can select a suitable extraction method without further ado depending on the nature of the sample to be tested.

If a sample containing RNA is to be used as a template for an amplification reaction, it will be necessary to transcribe said RNA in cDNA before amplification can be carried out. Again, techniques for doing so are well known to the person skilled in the art. As an example the RNA may be purified with RNeasy^{™} Mini Kit (Qiagen). The RNA will then be reversely transcribed to cDNA using, e.g. the SuperScript^{™} Choice System (Invitrogen).

In a further embodiment of the present invention a method is provided for determining whether a bovine is affected with the Cholesterol Deficiency syndrome or carrier of Cholesterol Deficiency by analyzing its DNA, the method comprising the steps of:
a. obtaining a sample of material containing said genomic DNA from the bovine,
b. extracting the DNA from said sample,
c. genotyping said DNA for genetic markers that are linked to the CD locus
d. ascertaining whether said animal carries the CD mutation by linkage analysis.

The term "genetic markers linked to the CD locus" refers to DNA sequence variants such as microsatellite markers or Single Nucleotide Markers (SNPs) that are located on bovine chromosomes 11 at less than 50% genetic recombination units from the CD locus. In the bovine, 50% genetic recombination units corresponds to approximately 50 million base pairs.

The term "ascertaining whether said animal carries the CD mutation by linkage analysis", refers to the determination of which allele at any of the genetic markers linked to the CD locus is associated with the CD mutation in a known carrier parent (which can be either the sire, the dam or both), and determining whether such linked marker allele is transmitted to the tested individual using standard linkage analysis procedures which are well known by those skilled in the art. Standard linkage analysis procedures are inter alia referred to in (8).

In another embodiment of the present invention a method is provided for determining whether a bovine is affected with the Cholesterol Deficiency syndrome or a carrier of Cholesterol Deficiency by analyzing its DNA, the method comprising the steps of:
a. obtaining a sample of material containing said genomic DNA from the bovine,
b. extracting the DNA from said sample,
c. genotyping said DNA for genetic markers that are in linkage disequilibrium with the CD locus,
d. ascertaining whether said animal carries the CD mutation by association analysis.

The term "genetic markers that are in linkage disequilibrium with the CD locus" refers to DNA sequence variants such as microsatellite markers or Single Nucleotide Markers (SNPs) that are in linkage disequilibrium with the CD locus in cattle populations. Linkage disequilibrium, also referred to as gametic association or association, refers to the non-random assortment of alleles at distinct genetic loci in the general population. In the present case, these are DNA sequence variants for which one allele is more often associated with the CD mutation in the general population than expected only by chance. In the bovine these include genetic markers, whether microsatellites or SNPs, that are located between nucleotide positions 74 million and 83 million on bovine chromosome 11 (bosTau6 assembly).

The term "ascertaining whether said animal carries the CD mutation by association analysis" indicates that one will determine whether said animal is carrier of the CD mutation from the analysis of its genotype at DNA sequence variants that are in linkage disequilibrium with the CD mutation. The association analysis can be performed by extracting linkage disequilibrium information from DNA sequence variants considered individually ("single point analyses"), or by considering the DNA sequence variants jointly ("multipoint analyses" including "haplotype-based analyses"). The principles of association studies are known by those skilled in the art and are for instance described in (9).

### Brief description of the figures

**Figure 1****: *APOB* insertional mutation.** IGV (Integrative Genome Viewer) screen capture of the BTA11 *APOB* exon 5 'BAM' (alignment) files for a carrier and a non-carrier animal, highlighting the discordant paired-reads characterizing the *APOB* insertional mutation in the carrier animal.
**Figure 2****: Target site duplication signature of the insertional breakpoint.** IGV screen capture of the BTA11 *APOB* exon 5 'BAM' files for a carrier animal allowing the breakpoint definition at single-base pair resolution and showing a 6 bp target-site duplication (blue triangle).
**Figure 3****: PCR-based diagnostic test for the *APOB* mutation.** A. Schematic representation of the PCR-based test developed to allow carrier identification. B. PCR amplicons separation by QIAxcel automated capillary electrophoresis for the three *APOB* genotypes.
**Figure 4****: Full-length BoERVK insertion in *APOB* exon 5.** A. Schematic representation of the BoERVK element inserted in *APOB*; TDS for Target site duplication. B. IGV screen capture of the MinION and MiSeq sequence reads mapped on a deduced consensus sequence. C. BoERVK full-length sequence, the TDS is underlined and the identical 5' and 3' LTR are shown in blue.
**Figure 5****: Transcriptional termination of the mutant *APOB* mRNA**. IGV screen capture of RNASeq data from calf livers (mutant and wild-type), revealing the transcriptional termination of *APOB* mutant mRNA at the insertion.

### Detailed description of the invention

### Development of a diagnostic test for Cholesterol Deficiency

A PCR-based diagnostic test combining one forward (i) and two reverse (ii)(iii) primers, respectively located in (i) *APOB* intron 4 (test_UP1: 5'- AAG GAG GCT GCA AAG CCA CCT AG -3'), (ii) inserted sequence upstream breakpoint (test_DN1, mutant allele specific: 5'- CCT TTG TCA CGA GTG GAA TGC CT-3') and (iii) *APOB* exon 5, downstream of the insertion (test_DN2: 5'- CCT CTT GAT GTT GAG GAT GTG TT -3'), was developed (Figure 3). Briefly, after an initial denaturation at 95°C for 2min, the PCR reaction was carried out in a 25□l final volume (1ng/□L of genomic DNA; 0.5□M of primers; 0.625U of GoTaq (Promega); 1X PCR buffer, 0.2mM dNTP) for 35 cycles (95°C 45sec; 55°C 40sec; 72°C 40sec). PCR fragment analysis was performed with the QIAxcel Advanced System (Qiagen).

### References

1. Shuster DE, Kehrli ME Jr, Ackermann MR, Gilbert RO. (1992) Identification and prevalence of a genetic defect that causes leukocyte adhesion deficiency in Holstein cattle. Proc Natl Acad Sci U S A. 89:9225-9229.
2. Thomsen B, Horn P, Panitz F, Bendixen E, Petersen AH, Holm LE, Nielsen VH, Agerholm JS, Arnbjerg J, Bendixen C. (2006) A missense mutation in the bovine SLC35A3 gene, encoding a UDP-N-acetylglucosamine transporter, causes complex vertebral malformation. Genome Res. 16:97-105. Epub 2005 Dec 12.
3. Charlier C, Coppieters W, Rollin F, Desmecht D, Agerholm JS, Cambisano N, Carta E, Dardano S, Dive M, Fasquelle C, Frennet JC, Hanset R, Hubin X, Jorgensen C, Karim L, Kent M, Harvey K, Pearce BR, Simon P, Tama N, Nie H, Vandeputte S, Lien S, Longeri M, Fredholm M, Harvey RJ, Georges M. (2008) Highly effective SNP-based association mapping and management of recessive defects in livestock. Nat Genet. 40:449-54. Epub 2008 Mar 16.
4. Kipp S, Segelke D, Schierenbeck S, Reinhardt F, Reents R, Wurmser C, Pausch H, Fries R, Thaller G, Tetens J, Pott J, Piechotta M, Grünberg W (2015) A new Holstein Haploytpe affecting calf survival. Interbull presentation
5. McLaren W., Pritchard B., Rios D., Chen Y., Flicek P., Cunningham F. (2011) Deriving the consequences of genomic variants with the Ensembl API and SNP Effect Predictor. Bioinformatics 26, 2069-70
6. Garcia-Etxebarria K, Jugo BM (2010) Genome-wide detection and characterization of endogenous retroviruses in Bos taurus. J Virol. 84(20):10852-62.
7. Syvanen, A.C. (2001) Accessing genetic variation: genotyping single nucleotide polymorphisms. Nature Reviews Genetics 2: 930-942.
8. Ott J. (1999). Analysis of Human Genetic Linkage. Third edition. Johns Hopkings University Press.
9. Ioannidis JP, Thomas G, Daly MJ (2009). Validating, augmenting and refining genome-wide association signals. Nat Rev Genet., 10(5):318-29.

## Claims

1. A method for determining whether a bovine is affected by Cholesterol Deficiency or a carrier of Cholesterol Deficiency by analyzing its genomic DNA, the method comprising the steps of:
a. obtaining a sample of material containing said genomic DNA from the bovine,
b. extracting the DNA from said sample,
c. genotyping said DNA for the CD mutation, i.e. a 7.5 Kb insertion of a BoERV element in exon 5 of the APOB gene, and
d. determining whether said animal carries the CD mutation.

2. A method for determining whether a bovine is affected by or a carrier of Cholesterol Deficiency by analyzing its RNA, the method comprising the steps of :
a. obtaining a sample of biological material containing said RNA from the bovine,
b. extracting the RNA from said sample,
c. genotyping said DNA for the CD mutation, i.e. a 7 Kb insertion of a BoERV element in exon 5 of the APOB gene, and
d. determining whether said animal carries the CD mutation.

3. A method for determining whether a bovine is a carrier of Cholesterol Deficiency by analyzing its DNA, the method comprising the steps of:
a. obtaining a sample of material containing said genomic DNA from the bovine,
b. extracting the DNA from said sample,
c. genotyping said DNA for genetic markers that are linked to the CD locus,
d. ascertaining whether said animal carries the CD mutation by linkage analysis.

4. A method for determining whether a bovine is a carrier of Cholesterol Deficiency by analyzing its DNA, the method comprising the steps of:
a. obtaining a sample of material containing said genomic DNA from the bovine,
b. extracting the DNA from said sample,
c. genotyping said DNA for genetic markers that are in linkage disequilibrium with the CD locus,
d. ascertaining whether said animal carries the CD mutation by association analysis.

5. The method of any one of claims 1 to 4, wherein the sample is selected from the group consisting of blood, sperm, hair roots, milk, body fluids and/or tissues including nucleated cells.

6. The method of any one of claims 1 to 5, wherein the bovine is selected from the species bos taurus.

7. The method of claim 6 wherein the bovine is selected from the group consisting of Holstein, Friesian and Holstein-Friesian Cross breeds, British and /or Dutch Friesian.

8. Use of the method of any one of claims 1 to 7 to perform marker assisted selection or genomic selection for increased survival in said bovine.


SEQUENCE LISTING

<110> Universite de Liege

<120> DETECTING CHOLESTEROL DEFICIENCY MUTATION IN CATTLE

<160> 1

<170> BiSSAP 1.2

<210> 1
<211> 6943
<212> DNA
<213> Bos taurus

<220>
<221> source
<222> 1..6943
<223> /organism="Bos taurus"
      /mol_type="genomic DNA"

<400> 1
gagccatagg ctaggtggct ttgcagccac ctagcctatg gctcacggta gagaagggac      60

cctcggtgac catcctctct ctgcaccagg tacgacctca agctggctgt tcctgaaggt     120

gcggggagcc ggtgaggcat tccactcgtg acaaaggtca tgaggaagga ggctcggcat     180

acgcaaaggc gggatcgagc ctcaggagtc cccccggata ttctcgagca ttttccccca     240

aaaaaccaga gtctgcctac tttattgctt tgtgctctca cctctgactt tactgggggc     300

tgtcccctac caccgtctct ctctctctgt gtcaaagagt taacttacag ctccaattaa     360

taaagttcct gggcaattag gagtgtttaa atccaaaccc ctctgatggc tctctaactc     420

gcctgacaag tttacccgga ctcctgcagc tatgcatacg attgtttaca gtctcccagc     480

ctcgagaggc atgggaagct taagatattc aaatagctta gagcctctca gagagttaaa     540

aactgtcaga ataaactagt aaaggatttc attgatgagt caatgcttgt tgccaagttt     600

tcacatcccc tgaattgtat ccttgaatat gtatcaatta atagtgggta tgtagaaaaa     660

ataagtagtg gccttggtgt tagtaacttt agacccttaa ggtaataaat tctttctttg     720

ttgtaaaccc attacacatc cgccctatag gaatgcaatt ttatctttgg aagatggtgc     780

caaaccttga aataattact cttagagaaa gtaagtcttt gttgataagt ccttgtcaag     840

agtcataaaa tgttagtagg ccttctggcc agaagatgat gtaaatcacc taaaccattt     900

gtatacgata catttgcagg aaagaaacct tggtttttga taagaatcaa agactgctga     960

ctttgcatcc cctattatcc tctatgtgta acttagggta taaaagcccc tgttaaaaat    1020

aaagctacgg gccttgctca ccaacgcttg gtctccccat gtcattcttt taacttccag    1080

ctgagtctcc atctggagcg cggaacccac cacgcttact aatcatgcct gggcttctaa    1140

gacccactcg agaaggtgtc tagggtgaga caccttccgc tattcgagag ggcgcctgcg    1200

gcctacgtaa gtggtgcaaa cttcttgtct tgaagtttta ttggtctccc gcgtaaacca    1260

agctactcag cttcttttct ccactgaaat ttcctactga gctatcctca ttctattgtt 1320

ctctatatcc ctaattagca tataaatagt cgccgacgcc gtctcccctt cgaataccct 1380

ggatcagccg gggctggtcc tcggcaggtg gcgcccgata cagggatttc gaaggtaagt 1440

ccccaacccc attcccccag tgttgagttt tcgggacgga taggacccca cttagggtgc 1500

tgcagacccc cctgtagaat agacagggag agaggagtgg gaagtgttga aagtgttgaa 1560

gagttagaga gaaaaaacga tttctaaaaa ggctgacaaa aaaggcctaa tcttttgata 1620

gctaaaagca aaatctttta ctatacttaa ttttctgaca tgggtaatac tgaatcaaat 1680

gaaagacagc tctttatagg agtaatttta cagttattag gtaaaagagg aattaaagtt 1740

aaaaaatctg ccattcaatc atttctttca tttgtacaag agcactgtcc ctggtttcca 1800

gacgaaggct ctgttaactt agatgtctgg gaaaaagtag gaaaacagtt aaaaacttac 1860

catgcagaac atggctcaga aaaggtgcct aatgacgcct tttccttatg gaatattatt 1920

agagatgtct tagaccctgc cccagattca gaaaaagtac atcttaaaag ggatagtgaa 1980

gaaaatgctg tagttaaacc tacccctgaa cctaaaaaag taacctttaa agaggaaaat 2040

gaagtcgaaa tcgtagttaa acctgaagaa aatgagaaaa acgaagaccc gcctgactat 2100

cggcaactaa gaaaaatgtt aatagccgtg actactcagg aacaacctaa agatagggat 2160

gaggaacaag atcagccttc cccaaaacaa aaagagaatt taggcgagat aacaactaaa 2220

tatcactctg atggagattg gcatctctta aacaaagatg ccccaaaagg cctaagagaa 2280

acttcccctg tcagaccatc cgctcctagg agcttgaggg aaacatcccc gatcagacag 2340

tttgtaagat ttagtcacca aacagttaaa ggatctccgg agcaggagaa gagaaatagg 2400

ggacgctccc atccctccat gcctcctcct ctgtgtgggg gtaaagggcc tccactagga 2460

gtttccccga gaagggtttg ctctagtccc aaggataaat ttgatcctca ccttgaggct 2520

tcttttctag tcccctctgc agtttagata gggtaaagaa ggtacaaaat aaataaaaat 2580

attaaaaaat aaaaaaaaaa tgggtttctc tgcatctaag aatatacaaa tgtttattgg 2640

ctctgtgaaa tatgattaga aatgtcctgg acccctctca tgaggctgtt aaatagcctt 2700

tgagggaggc tatagcggta gatggtggtg agtctccagt ttctgcatag atcataattt 2760

ctgctattgg aaaaaaaaaa aaaaaaggag ggagaaagtg ctttacctcc cgagcaagga 2820

gagggtttac aggacgctgc ggccgagcgc cctggcgagc tccctccccc tctgcgcaaa 2880

cctttaaaaa acttatccac cactttctga tttaaggtga tcgccaccgc ctccgtttgt 2940

gcctcccagg acccaggagt tccccacttt gcccccaaag cctctcaaag cgttgcctaa 3000

gcctgaggaa gataaaaagt tttttgaaca gtggagcttt taaaacagac tgcgtgcaca 3060

atatgcagag catgcttctt agagaaaacc ccctcagggg gggtctcacc caggctagga 3120

gaaaagcgaa accgcaaggg gatttagcaa caatattaac ccctgatgtg ccgcttactc 3180

caattccaac gggagtggct ggccccctac ctttggacat tgtaagatct gtcctggggc 3240

atagctcgct ttcttttcaa aaaaaaaaaa aaaaaaaaat tcggtggtgc atggtgtagt 3300

agattctgat tatattgaga aattaaagtc ttgatatcac cgcctaccaa aactgtacaa 3360

attaataaag gtcaaagagt aacacagcct ttgcttttac cttattatca gacaagaaaa 3420

aacttgactt ctcaagttaa gagccacgaa acatttggat ctagtgatct agccttttgg 3480

atgcaggaaa ttacagctcc tggccaacac atactactga aaatgagttg gtgggattag 3540

agaaagtggt atgtgaggtg gggatgctat acaggtggta catttaacaa tactttatat 3600

attgttataa atacataata taaatatatt tgcctaatta cagtttgcct aattaggtat 3660

gggtataaat aaaatataat aaaggtatac ctaattctac ttatagatct atacttaatt 3720

tgtatataaa ttaatatgta tactatatat acatatatat atactgtata attaaatgta 3780

tattgcagta atataatgtg tgtggctgat attaattggt atggattggt gtgctgtgat 3840

gatatatgtt ctatatactg tataattgta tatgtgtgac atgcattatt gcagtacatt 3900

ggtttgtgtt ggttggtatt agctgtgtat gtgttgtatt gctgtaatat atattaatta 3960

atatattaat tataaagatt aattcaaata tattgattta tatatattat atgtcaataa 4020

gtttatactt gttgccatat ataaatacat atataaatac attttacatt taggtatgtc 4080

tgtataccaa aatgagataa ggaggttata catttattac ttaaatttat acagagacct 4140

aaactaaaca ttagacctaa attaacatgt ccctaggaaa acagctggac aaatagtaaa 4200

gttatgtcca aattgtaatt aatcacttaa aggtaaacac acaggggcag actacaatga 4260

tgagacacta gatgcctgaa ttacaggcta cgactcggcc cctggtcaag taaaaagacc 4320

tccttactgg agagtgtaaa aggccagatg tgttgctaac ttgtgggaga gggtatgctt 4380

atatatttct acagaatgca gattctccga tttggatctc agaaaaattc gtcatgtcac 4440

tttccccaaa gaccaggttc gcggcgcgcg cactcgctcg ctccccctcc cccagccagc 4500

tctctccgcc gccagcggcg ccctgcaccc tccttgctgc accccgagac ctagagcaaa 4560

gaagtctgtg cggcgagtga gggccagaga ggaaagcgcg cccgcgcagt gcagtccaga 4620

ccagcgcgct cccgccgcgg gcgacaggaa aacgccccga gcccaagcgg cgcggctagc 4680

ccgagtccgc gaaccccgcc cctccgcccg ccgtagacgc ctcggcccac aacctgccgt 4740

tcgcgttctg cctcctgatg ctgcggacac tgctccgccg agccgatgcc tacagctgct 4800

ccttgtctca tcttgtttaa atcctcaata taatgtttgc tcctttgtaa tgttacaatg 4860

tccaccttat cttatgatgt aaccacttat tgaattatgc tcttgctgta ttacaacaac 4920

tgcaggattt aatgcgaaca cgacggtttg tgggcttact tatcttggga atagcagctt 4980

tgataagcgc aattacttct gttactgtgg cagcaatatc attgactcaa caagtacata 5040

ctgctcaata tgttgattct atgtccaaaa atgtttcttt agcattggca acacaggaag 5100

ctatagacag gaaattagaa atgagggtag atgccctaga ggaagcagta atacatattg 5160

ggactgaatt gcaggcttta aaggtgaaaa tggcattgtc ctgtcatgct gactaccggt 5220

ggatatgtgt aacacccctg aaagtaaatg agacagattt tgaatgggaa aagattaaaa 5280

accatatttc aggtatttgg aacagctctg acattagctt agacttaggg aaacttcaca 5340

atcaaatagc aaccctggaa cactcccgat tagattttac tgccgctgga acagcaaatg 5400

atttcttcca tactttctct aactacattt caggaaaaaa tattctgtct accttcctcg 5460

gctacgctac cttggctgtt ttaattttat ttctaataat cattcttcct tgtattgtca 5520

ggattcttcg gcagagcatt cagaggctcg cgactgagct acatctggct gttttaagaa 5580

ataaaaaagg gggagatgcg gggagccggt gaggcattcc actcgtgaca aaggtcatga 5640

ggaaggaggc tcggcatacg caaaggcggg atcgagcctc aggagtcccc ccggatattc 5700

tcgagcattt tcccccaaaa aaccagagtc tgcctacttt attgctttgt gctctcacct 5760

ctgactttac tgggggctgt cccctaccac cgtctctctc tctctgtgtc aaagagttaa 5820

cttacagctc caattaataa agttcctggg caattaggag tgtttaaatc caaacccctc 5880

tgatggctct ctaactcgcc tgacaagttt acccggactc ctgcagctat gcatacgatt 5940

gtttacagtc tcccagcctc gagaggcatg ggaagcttaa gatattcaaa tagcttagag 6000

cctctcagag agttaaaaac tgtcagaata aactagtaaa ggatttcatt gatgagtcaa 6060

tgcttgttgc caagttttca catcccctga attgtatcct tgaatatgta tcaattaata 6120

gtgggtatgt agaaaaaata agtagtggcc ttggtgttag taactttaga cccttaaggt 6180

aataaattct ttctttgttg taaacccatt acacatccgc cctataggaa tgcaatttta 6240

tctttggaag atggtgccaa accttgaaat aattactctt agagaaagta agtctttgtt 6300

gataagtcct tgtcaagagt cataaaatgt tagtaggcct tctggccaga agatgatgta 6360

aatcacctaa accatttgta tacgatacat ttgcaggaaa gaaaccttgg tttttgataa 6420

gaatcaaaga ctgctgactt tgcatcccct attatcctct atgtgtaact tagggtataa 6480

aagcccctgt taaaaataaa gctacgggcc ttgctcacca acgcttggtc tccccatgtc 6540

attcttttaa cttccagctg agtctccatc tggagcgcgg aacccaccac gcttactaat 6600

catgcctggg cttctaagac ccactcgaga aggtgtctag ggtgagacac cttccgctat 6660

tcgagagggc gcctgcggcc tacgtaagtg gtgcaaactt cttgtcttga agttttattg 6720

gtctcccgcg taaaccaagc tactcagctt cttttctcca ctgaaatttc ctactgagct 6780

atcctcattc tattgttctc tatatcccta attagcatat aaatagtcgc cgacgccgtc 6840

tccccttcga ataccctgga tcagccgggg ctggtcctcg gcatgaaggc aagcaagttc 6900

tactttaccc agagaaagaa gagcctaaac acagcctcaa cat 6943